(19) Europäisches Patentamt
European Patent Office
Office européen des brevets

(11) Veröffentlichungsnummer : **0 137 294**
**B1**

(12) # EUROPÄISCHE PATENTSCHRIFT

(45) Veröffentlichungstag der Patentschrift : **26.04.89**

(51) Int. Cl.[4] : **A 61 K 37/02**

(21) Anmeldenummer : **84110442.5**

(22) Anmeldetag : **03.09.84**

(54) **Gonadoliberin und Gonadoliberinagonisten zur Behandlung von Stoffwechselerkrankungen, die auf zu geringe Parathormonspiegel zurückzuführen sind.**

(30) Priorität : **08.09.83 DE 3332329**

(43) Veröffentlichungstag der Anmeldung : **17.04.85 Patentblatt 85/16**

(45) Bekanntmachung des Hinweises auf die Patenterteilung : **26.04.89 Patentblatt 89/17**

(84) Benannte Vertragsstaaten : **AT BE CH DE FR GB IT LI LU NL SE**

(56) Entgegenhaltungen :
**FR-A- 2 450 109**
**FR-A- 2 465 486**

(73) Patentinhaber : **HOECHST AKTIENGESELLSCHAFT**
**Postfach 80 03 20**
**D-6230 Frankfurt am Main 80 (DE)**

(72) Erfinder : **König, Wolfgang, Dr.**
**Eppsteiner Strasse 25**
**D-6238 Hofheim am Taunus (DE)**
Erfinder : **Neubauer, Horst, Dr.**
**Am Eichkopf 12**
**D-6240 Königstein/Taunus (DE)**

**EP 0 137 294 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Parathormon (PTH) reguliert zusammen mit Calcitonin (CT) den $Ca^{2+}$-Haushalt. Ist der $Ca^{2+}$-Spiegel zu tief, so wird die Bildung des PTH stimuliert, das den $Ca^{2+}$-Spiegel erhöht (The New England J. Med. 307, 1982 Seite 226-228). PTH greift hierbei direkt an den Knochen an und mobilisiert $Ca^{2+}$. Calcitonin senkt dagegen den $Ca^{2+}$-Spiegel.

Die Regulierung des Plasma-$Ca^{2+}$ ist von großer Bedeutung. Z. B. ist der Bluthochdruck offensichtlich auch $Ca^{2+}$-abhängig. So senken $Ca^{2+}$-Antagonisten den Blutdruck. Hypertensive Patienten haben einen geringeren Serumspiegel an ionisierten $Ca^{2+}$, besitzen dafür aber mehr zellmembrangebundenes $Ca^{2+}$ als Normotensive (Science 217, 1982, Seite 267-269). Der $Ca^{2+}$-Influx in die Zelle ist also erleichtert und es kommt leichter zu Kontraktionen der glatten Muskulatur, was letztendlich zu einer Erhöhung des Blutdrucks führt. Bei spontan hypertensiven Ratten konnte man feststellen, daß PTH eine blutdrucksenkende Wirkung hat (1982 Blood pressure council, Supp. I, Hypertension 5, 1983, S. 59-63). Auch an intakten Tieren hat PTH einen blutdrucksenkenden Effekt. In in vitro-Testen zeigte sich an vaskulären Gefäßen eine vasodilatatorische Wirkung (Adv. Exp. Med. Biol. 151, 1982, Seite 619-632).

Ein Zuwenig an PTH kann also zu einem geringen Serum $Ca^{2+}$-Spiegel führen, der wiederum durch eine Vermehrung der $Ca^{2+}$-Rezeptoren an den Zellmembranen einen erhöhten Blutdruck bewirken kann. Rein theoretisch könnte auch ein Zuwenig an $Ca^{2+}$ einen zu niedrigen Blutdruck bewirken, wenn die Zellmembranen nicht vermehrt $Ca^{2+}$-Rezeptoren ausbilden.

Über die Freisetzung von Glukagon hat PTH auch einen Einfluß auf den Blutzuckerspiegel. PTH erhöht die Glykogenbildung aus Glucose (FEBS Lett. 148, 1982, Seite 31-34), stimuliert dagegen auch die Freisetzung von Glukagon und kann damit auch hyperglykämisch wirken (Clin. Res. 28, 1980, Seite 55 A). Es hat also einen regulierenden Einfluß auf den Blutzuckerspiegel. Bei Muskelschmerzen, die auf eine Unterversorgung von Blutglucose zurückzuführen sind (McArdle-Erkrankung) schafft z. B. eine Glukagontherapie Erleichterung (Dtsch. Med. Wochenschr. 107, 1982, Seite 1809-1811).

Substanzen, die zur Stimulierung von PTH befähigt sind, können also therapeutisch zur Regulierung des $Ca^{2+}$-beding ten Bluthochdrucks oder zur Erhöhung der Blutglucose, wie z. B. bei der McArdle-Erkrankung, eingesetzt werden.

Es wurde gefunden, daß Gonadoliberin und dessen agonistisch wirkenden Analoga überraschenderweise in kleiner Dosierung PTH stimulieren. Bereits Dosen von 5 ng/kg Buserelin oder 50 ng/kg Gonadoliberin stimulieren bei Ratten das PTH.

Die Erfindung betrifft daher pharmazeutische Zubereitungen zur parenteralen Verabreichung zur Behandlung von Stoffwechselerkrankungen, die durch zu geringe Sekretion endogenen Parathormons entstehen können, die gekennzeichnet sind durch einen Gehalt an 0,5 bis weniger als 5 µg Gonadoliberin/Einzeldosis oder 0,05 bis 0,5 µg eines Gonadoliberinagonisten/Einzeldosis für einen normalgewichtigen Erwachsenen oder, zur Verabreichung auf die Schleimhäute, durch einen Gehalt an 10 bis weniger als 50 µg Gonadoliberin/Einzeldosis oder 1 bis 20 µg eines Gonadoliberinagonisten/Einzeldosis für einen normalgewichtigen Erwachsenen in einem pharmazeutisch unbedenklichen Träger.

Stoffwechselerkrankungen, die durch zu geringe Sekretion endogenen Parathormons entstehen können, sind z. B. der Hypoparathyroidismus, die Hypocalcämie mit Tetanie, der Bluthochdruck als auch der zu niedrige Blutdruck, sowie Krankheiten, die auf zu geringe Glucoseversorgung beruhen.

Gonadoliberin ist ein Decapeptid der Formel 1, das im Hypothalamus gebildet wird und in der Hypophyse die Freisetzung von Follitropin und Lutropin bewirkt (Biochem. Biophys. Res. Commun. 43, 1971, Seite 1334).

$$\text{Glu—His—Trp—Ser—Tyr—Gly—Leu—Arg—Pro—Gly—NH}_2 \qquad (I)$$

Die diagnostische Dosis von Gonadoliberin zur Freisetzung der Gonadotropine (Follitropin und Lutropin) liegt bei einer parenteralen Applikation von 25 — 100 µg/erwachsener Mensch (Rote Liste 1983, 34002 und 24004). Die bei Kryptorchismus verwendete intranasale Dosierung bei Kindern zwischen zwei und sechs Jahren beträgt 3 mal täglich 400 µg (Rote Liste 1983, 49038).

Nach der FR-A-2 450 109 liegt die Dosis von LH-RH zur Regulierung der Zyklus bei einer parenteralen Verabreichung von 5-20 000 (intranasal : 50-200 000) µg/erwachsener Mensch.

Dagegen genügen beim erfindungsgemäßen Verfahren zur Behandlung von Stoffwechselerkrankungen, die auf zu geringe Parathormonspiegel zurückzuführen sind, parenterale Gaben von nur 0,5 bis weniger als 5 µg Gonadoliberin/normalgewichtigen Erwachsenen (≈6 bis weniger als 60 ng/kg/Dosierung). Bei Applikation auf die Schleimhaut, vorzugsweise bei intranasaler Applikation genügen Dosierungen von nur 10 bis weniger als 50 µg Gonadoliberin/normalgewichtigen Erwachsenen (≈0,1 bis weniger als 0,7 µg/kg/Dosierung). Eine starke Beeinflußung der Gonadotropine ist also während der Behandlung mit niedrig dosiertem Gonadoliberin nicht zu erwarten. Die für die erfindungsgemäße Behandlung notwendige intranasale Dosis von 10 bis weniger als 50 µg/Mensch, die günstigstenfalls einer parenteralen Dosis von 0,5 bis 2,5 µg entspricht, liegt weit unter der für diagnostische Zwecke verwendeten Substanzmenge. Ensprechendes gilt für die erfindungsgemäße Behandlung mit Gonadoliberinagonisten (LH-RH-Analoga).

Die agonistische Wirkung des Gonadoliberins wird vor allem durch die Substitution von $Gly\text{-}NH_2^{10}$ mit dem Ethylamin-Rest und durch den Austausch von $Gly^6$ mit lipophilen D-Aminosäuren verstärkt. Man kommt so zu Verbindungen, die je nach Test etwa 100 — 200 mal stärker als Gonadoliberin wirken (Vitamins and Hormons 38, 1980, Seite 257-323). Wichtig für den Austausch in Position 6 ist, daß die D-Aminosäure eine α-CH-und eine β-$CH_2$-Gruppe enthält (Peptides — Chemistry, Structure, Biology, S. 883-888, Ann Arbor Science Publishers INC, Ann Arbor, Mich. 1975). Von den « natürlichen » D-Aminosäuren bewährten sich am besten D-Trp, D-Leu und der tert.-Butylether von D-Ser. Durch die Verwendung synthetischer aromatischer D-Aminosäuren lassen sich jedoch noch höhere Aktivitäten erzielen, wie z. B. bei [D-3-(2.4.6.Trimethylphenyl)-$Ala^6$]- und [D-3-(2-Naphthyl)-$Ala^6$] Gonadoliberin (J. Med. Chem. 25, 1982 Seite 795-801).

Infrage für die Indikation « Freisetzung endogenen PTHs » und der damit verbundenen Stoffwechselvorgänge kommen alle Gonadoliberinanaloga mit voller oder stärkerer Gonadoliberinwirkung. Geeignet sind beispielsweise folgende Analoga der Formel II :

$$\text{Glu-His-Trp-Ser-Tyr-X-Y-Arg-Pro-Z} \quad \text{(II)}$$

1 2 3 4 5 6 7 8 9 10

in welcher bedeutet (Abkürzungen siehe Houben-Weyl, Methoden der organischen Chemie, 4. Aufl., Bd. XV/1 und XV/2, Thieme Verlag Stuttgart) :

a) Z = $Gly\text{-}NH_2$,
Y = Leu und
X = D-Nle, D-Nva, D-Abu, D-Phe, D-Ser, D-Met, D-Pgl, D-Lys, D-Leu, D-Arg, D-Ser($Bu^t$), D-Thr($Bu^t$), D-Cys($Bu^t$), D-Lys, D-Asp($OBu^t$), D-Glu($OBu^t$), D-Orn(Boc), D-Lys(Boc), D-Trp, D-Tyr, ε-Lauryl-D-Lys oder ε-Dextran-D-Lys, D-His(Bzl)
oder
b) Z = $Gly\text{-}NH_2$, NH-($C_1$-$C_3$)-Alkyl bzw. NH-Cyclopropyl, die durch OH oder F substituiert sein können,
Y = Leu, Ser($Bu^t$), Cys($Bu^t$), Asp($OBu^t$), Glu($OBu^t$), Orn(Boc) oder Lys(Boc) und
X = D-Ser($Bu^t$), D-Cys($Bu^t$), D-Asp($OBu^t$),
D-Glu($OBu^t$), D-Orn(Boc) oder
D-Lys(Boc), D-His(Bzl)
wobei $Ser^4$ gegebenenfalls durch Ala oder Thr, Tyr gegebenenfalls durch Phe und Arg gegebenenfalls durch Orn, Lys oder Homoarginin ersetzt ist, oder
c) Z = $—NHCH_3$, $—NH—CH_2—CH_3$, $—NH—CH_2CH_2CH_3$,

$-NH-CH_2CH_2-OH$, 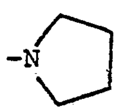 oder 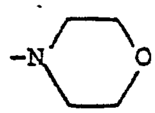

Y = Leu und
X = Gly
oder
d) Z = $—NHC_2H_5$,
Y = Leu und
X = D-Trp, D-Leu, D-Ala, D-Ser($Bu^t$), D-Tyr, D-Lys oder D-His(Bzl)
oder
e) Z = $Gly—NH_2$ oder $NH—C_2H_5$,
Y = N-α-Methyl-Leu und
X = Gly
oder
f) Z = NH-Cyclopropyl,
Y = Leu
X = D-Leu
oder
g) Z = $Gly\text{-}NH_2$, NH-($C_1$-$C_3$)-Alkyl oder NH-Cylopropyl,
Y = Ser($Bu^t$), Cys($Bu^t$), Asp($Obu^t$), Glu($OBu^t$), Orn(Boc) oder Lys(Boc) und
X = Gly.

Verbindungen der Formel II sowie Verfahren zu deren Herstellung sind beispielsweise bekannt aus :

Coy D.H., Labrie F., Favary M., Coy E.J. et Schally A.V. (1975) LH — releasing activity of patent LH — RH analogs in vitro BBRC 67, 576-582.

Wylie Vale et al. (1976) in « Hypothalamus and endocrine functions » (F. Labrie, J. Meites and G. Pelletier, eds ; Plenum Press) pages 397-429.
Fujino, BBRC vol. 49, n° 3, 1972, page 863.
DE-A-2 509 783, US-A-3 901 872, US-A-3 896, 104,
JP-A-4 9100-081, US-A-3 971 737, BE-A-842 857,
BE-A-832 310, BE-A-832 311, US-A-4 003 884,
US-A-4 024 248, DE-A-27 20 245 und DE-A-24 46 005.

Als besonders geeignet haben sich beispielsweise folgende Analoga erwiesen :

[D-Ser(Bu$^t$)$^6$] Gonadoliberin-(1-9)-nonapeptid-ethylamid (=Buserelin)
(Drugs of the Future 4, 1979, Seite 173-177, 8, 1983 S. 254),
[D-Trp$^6$] Gonadoliberin (Drugs of Future 3, 1978, S. 645-646),
[D-Trp$^6$] Gonadoliberin-(1-9)-nonapeptid-ethylamid (Drugs of the Future 7, 1982, Seite 637-642),
[D-Leu$^6$] Gonadoliberin-(1-9)-nonapeptid-ethylamid (Drugs of the Future 7, 1982, Seite 882-886),
[D-Ser(Bu$^t$)$^6$, AzaGly$^{10}$] Gonadoliberin (Drugs of the Future 5, 1980, Seite 191-192 ; 1983, Seite 364-365),
[D-Trp$^6$, N-MeLeu$^7$] Gonadoliberin-(1-9)-nonapeptid-ethylamid (Drugs of the Future 8, 1983, Seite 347-350),
[D-α-Aminoadipinsäure-δ-tert.butylester$^6$] Gonadoliberin-(1-9)-nonapeptid-ethylamid (DE-A-30 20 941) und die oben einleitend aufgezählten Analoga mit den unnatürlichen Aminosäuren.

Die hier im einzelnen aufgezählten LH-RH-Analoga sind alle hochwirksam. Sie wirken am Menschen etwa 10 mal stärker als Gonadoliberin und haben eine längere Wirkungsdauer. Sie müssen also in geringer Dosierung und nicht so oft wie Gonadoliberin dosiert werden. Gewöhnlich genügen bei parenteraler Gabe 50 bis 500 ng der o. g. Gonadoliberinagonisten/normalgewichtigen Erwachsenen ($\approx$ 0,6 bis 70 ng/kg/Dosis). Bei Applikation auf die Schleimhäute (z. B. intranasale Gabe) werden 1-20 µg Analogen/normalgewichtigen Erwachsenen ( $\approx$ 10. bis 300 ng/kg/Dosis) notwendig. Es kann jedoch auch notwendig werden, bei besonders stark wirksamen LH-RH-Analoga, die Dosis zu erniedrigen, bzw. bei geringer wirksamen diese zu erhöhen.

In Ratten wurde durch eine $Ca^{2+}$-Gluconat-Infusion der Plasma-$Ca^{2+}$-Spiegel erhöht. Durch diese Infusion wird der PTH-Spiegel supprimiert (The New England J. Med. 307, 1982, S. 226-228). Eine Bolusinjektion der Gonadoliberinagonisten erhöhte jedoch signifikant den PTH-Spiegel gegenüber den Kontrollen und potenzierte das Plasma-$Ca^{2+}$. Das 30 Minuten nach der Injektion der Gonadoliberinagonisten erhöhte PTH bewirkte nach 60 Minuten einen signifikanten Serum-$Ca^{2+}$-Anstieg. Daß diese Beeinflußung des Plasma-$Ca^{2+}$-Spiegels durch endogenes PTH oder PTH selbst einen Einfluß auf den Blutdruck hat, wurde an einer Frau mit zu hohem Blutdruck überprüft. Nach einmaliger intranasaler Gabe von 100 mcg Gonadoliberin, was bei einer Resorptionsrate von 2-5 % also nur etwa 2-5 mcg resorbierten Gonadoliberins entspricht, konnte der erhöhte Blutdruck 8 Stunden lang stark gesenkt werden.

Der Einfluß auf den Blutzucker wurde zunächst an Kaninchen überprüft. Kaninchen wurden zur Hemmung des endogen Glucagons mit einem Glucosebolus versehen.

Nach Verabreichung von 5-50 ng/kg eines Gonadoliberinagonisten wurde der Plasma-Glucosespiegel signifikant erhöht, was auf eine Erhöhung des Glucagons beruht. Einer Patientin mit starken Muskelschmerzen am Oberarm (McArdle-Erkrankung) gab man 100 mcg Gonadoliberin intranasal. Die starken Dauerschmerzen verschwanden nach etwa 15 Minuten. Am Anfang der Behandlung wurde mehrmals täglich (3 mal), später 2 mal und dann nur noch einmal täglich appliziert um einer Tachyphylaxie vorzubeugen. Im Laufe der Therapie konnte der Arm wieder ohne Schmerzen bewegt werden. Der anfangs sehr schwache Blutdruck der Patientin von 90/60 wurde interessanterweise nicht weiter gesenkt, sondern leicht erhöht, so daß er sich während der Therapie auf 110/65 einpendelte. Diese kleinen Gonadoliberindosen wirken also nicht generell blutdrucksenkend, sondern können auch bei niedrigem Blutdruck blutdruckerhöhend wirken.

Gonadoliberin und seine Agonisten bewirken also z. B. über die Freisetzung von endogenen PTH eine Regulierung des Blutdrucks und des Blutzuckers.

Die erfindungsgemäßen Zubereitungen können intranasal oder parenteral verabreicht werden. Für eine intranasale Anwendungsform werden die Wirkstoffe mit den dafür üblichen Zusatzstoffen wie Stabilisatoren oder inerten Verdünnungsmitteln vermischt und durch übliche Methoden in geeignete Darreichungsformen gebracht, wie wäßrige, alkoholische oder ölige Suspensionen oder wäßrige, alkoholische oder ölige Lösungen. Als ölige Trägerstoffe oder Lösungsmittel kommen beispielsweise pflanzliche oder tierische Öle in Betracht, wie Sonnenblumenöl oder Lebertran.

Zur subkutanen oder intravenösen Applikation werden die aktiven Verbindungen oder deren physiologisch verträglichen Salze, gewünschtenfalls mit den dafür üblichen Substanzen wie Lösungsvermittler, Emulgatoren oder weitere Hilfsstoffe in Lösung, Suspension oder Emulsion gebracht. Als Lösungsmittel kommen dafür z. B. in Frage : Wasser, physiologische Kochsalzlösungen oder Alkohole, z. B. Ethanol, Propandiol oder Glycerin daneben auch Zuckerlösungen wie Glucose- oder Mannitlösungen, oder auch eine Mischung aus den verschiedenen genannten Lösungsmitteln.

Zur weiteren Erläuterung sollen die folgenden Beispiele dienen, ohne die Erfindung auf diese zu beschränken :

Beispiel 1

Stimulierung der Plasma-Spiegel von Parathormon und $Ca^{2+}$ durch Buserelin (siehe Fig. 1 und 2)

Zwei Gruppen von je 31 Wistarratten wurden mit Pentobarbital (45 mg/kg i.p.) narkotisiert. Man entnahm von allen Tieren eine Blutprobe (Ausgangswert). Anschließend gab man allen Tieren eine mindestens einstündige $Ca^{2+}$-Infusion (30 mg/kg/h, Applikationsvolumen 1,2 ml/h) und etwa 5 Minuten nach Infusionsbeginn der Verum-Gruppe eine Injektion von Buserelin (5 ng/kg i.v.) in physiologischer Kochsalzlösung mit 0,25 % Haemaccel®-Zusatz und der Placebo-Gruppe die gleiche Menge physiologische Kochsalzlösung mit 0,25 % Haemaccel®-Zusatz. Nach 30, 60, 90, 120 und 180 Minuten wurde ein Teil der Tiere getötet und entblutet. Das Blutserum wurde auf $Ca^{2+}$ und Parathormon untersucht. Nach 30 Minuten stieg Parathormon in der Verum-Gruppe im Vergleich zu der Placebogruppe signifikant an. Der durch die $Ca^{2+}$-Infusion bereits erhöhte $Ca^{2+}$-Spiegel war nach 60 Minuten in der Verum-Gruppe ebenfalls deutlich erhöht, was eine Folge des freigesetzten Parathormon ist.

Fig. 1 zeigt den zeitlichen Verlauf des PTH-Spiegels (pg/ml).

In Fig. 2 ist der zeitliche Verlauf des $Ca^{2+}$-Spiegels (mg %) zu sehen.

In beiden Figuren ist bei den einzelnen Meßpunkten die Anzahl n der Messungen sowie die Standardabweichung des Meßwertes (graphisch) angegeben.

Beispiel 2

Stimulierung der Plasma-Glucose durch Buserelin (siehe Fig. 3)

Je 3 wachen Kaninchen gab man einen Glucosebolus (500 mg/kg i.v.). Der Verum-Gruppe gab man gleichzeitig Buserelin (5 ng/kg i.v.) in physiologischer Kochsalzlösung mit 0.25 % Haemaccel®-Zusatz und der Placebo-Gruppe die gleiche Menge physiologische Kochsalzlösung mit 0.25 % Haemaccel®-Zusatz. Nach 10, 30, 40, 50, 60, 70, 80 und 90 Minuten wurde Blut abgenommen und daraus die Blutglucose bestimmt. In der Verum-Gruppe waren nach 70-90 Minuten die Blutglucosewerte signifikant erhöht.

Fig. 3 gibt den zeitlichen Verlauf der Blutglucosewerte beim Kaninchen wieder. Bei den einzelnen Meßpunkten ist jeweils graphisch die Standardabweichung des Meßwertes angegeben (n ist jeweils 3).

Beispiel 3

Blutdrucksenkung durch Gonadoliberin

Eine hypertensive, 48 Jahre alte Frau, deren Bluthochdruck mit β-Blockern behandelt wird, setzte ihre Behandlung am Versuchstag aus. Versuchsbeginn : 17.00 Uhr.

Der systolische Blutdruck von etwa 150 mm Hg sank nach intranasaler Gabe von 100 µg Gonadoliberin innerhalb von 30 Minuten auf etwa 125 mm Hg, blieb 90 Minuten bei 125 mm Hg und stieg dann für die nächsten 4 Stunden auf 130 mm Hg. Nach einer 8-stündigen Bettruhe, während der die Versuchsperson ausgezeichnet schlief, war der Blutdruck auf etwa 137 mm Hg gestiegen.

Beispiel 4

Behandlung von Muskelschmerzen

Eine Frau, 46 Jahre alt, hatte starke Schmerzen im Oberarm. Der Arm konnte nicht vom Körper weg bewegt werden. Nach einer intranasalen Applikation von 100 µg Gonadoliberin war der Dauerschmerz in Ruhehaltung verschwunden. Der sehr schwache Blutdruck von 90/60 stieg während der Behandlung mit Gonadoliberin auf 110/65. Am ersten Tag wurde insgesamt 3 mal, am zweiten und dritten Tag 2 mal und am vierten und folgenden Tagen nur noch einmal täglich am Morgen therapiert. Im Lauf der Therapie wurden auch die Bewegungsschmerzen weniger und der Arm konnte wieder normal bewegt werden.

**Patentansprüche** (für die Vertragsstaaten : BE, CH, DE, FR, GB, IT, LI, LU, NL, SE)

1. Pharmazeutische Zubereitung zur parenteralen Verabreichung zur Behandlung von Stoffwechselerkrankungen, die durch zu geringe Sekretion endogenen Parathormons entstehen können, gekennzeichnet durch einen Gehalt an 0,5 bis weniger als 5 µg Gonadoliberin/Einzeldosis oder 0,05 bis 0,5 µg eines Gonadoliberinagonisten/Einzeldosis für einen normalgewichtigen Erwachsenen in einem pharmazeutisch unbedenklichen Träger.

2. Zubereitung gemäß Anspruch 1 zur Behandlung von Hypoparathyreoidismus.

3. Zubereitung gemäß Anspruch 1 zur Behandlung von Hypocalcämie.

4. Zubereitung gemäß Anspruch 1 zur Normalisierung des Blutdrucks.

5. Zubereitung gemäß Anspruch 1 zur Behandlung von Krankheiten, die auf einer zu geringen Glucoseversorgung beruhen.

6. Pharmazeutische Zubereitung zur Verabreichung auf die Schleimhäute zur Behandlung von Stoffwechselerkrankungen, die durch zu geringe Sekretion endogenen Parathormons entstehen können, gekennzeichnet durch einen Gehalt an 10 bis weniger als 50 µg Gonadoliberin/Einzeldosis oder 1 bis 20 µg eines Gonadoliberinagonisten/Einzeldosis für einen normalgewichtigen Erwachsenen in einem pharmazeutisch unbedenklichen Träger.

7. Zubereitung gemäß Anspruch 6 zur Behandlung von Hypoparathyreoidismus.

8. Zubereitung gemäß Anspruch 6 zur Behandlung von Hypocalcämie.

9. Zubereitung gemäß Anspruch 6 zur Normalisierung des Blutdrucks.

10. Zubereitung gemäß Anspruch 6 zur Behandlung von Krankheiten, die auf einer zu geringen Glucoseversorgung beruhen.

11. Verfahren zur Herstellung einer Zubereitung gemäß einem der Ansprüche 1 bis 10, dadurch gekennzeichnet, daß man Gonadoliberin oder dessen Analogon zusammen mit einem pharmazeutisch unbedenklichen Träger in eine geeignete Zubereitungsform bringt.

**Patentansprüche** (für den Vertragsstaat AT)

1. Verfahren zur Herstellung einer pharmazeutischen Zubereitung zur parenteralen Anwendung bei der Behandlung von Stoffwechselerkrankungen, die durch zu geringe Sekretion endogenen Parathormons entstehen können, gekennzeichnet durch einen Gehalt an 0,5 bis weniger als 5 µg Gonadoliberin/Einzeldosis oder 0,05 bis 0,5 µg eines Gonadoliberinagonisten/Einzeldosis für einen normalgewichtigen Erwachsenen, dadurch gekennzeichnet, daß man Gonadoliberin oder dessen Analogon zusammen mit einem pharmazeutisch unbedenklichen Träger in eine geeignete Zubereitungsform bringt.

2. Verfahren zur Herstellung einer Zubereitung gemäß Anspruch 1 zur Anwendung bei der Behandlung von Hypoparathyreoidismus.

3. Verfahren zur Herstellung einer Zubereitung gemäß Anspruch 1 zur Anwendung bei der Behandlung von Hypocalcämie.

4. Verfahren zur Herstellung einer Zubereitung gemäß Anspruch 1 zur Anwendung bei der Normalisierung des Blutdrucks.

5. Verfahren zur Herstellung einer Zubereitung gemäß Anspruch 1 zur Anwendung bei der Behandlung von Krankheiten, die auf einer zu geringen Glucoseversorgung beruhen.

6. Verfahren zur Herstellung einer pharmazeutischen Zubereitung zur Anwendung auf die Schleimhäute bei der Behandlung von Stoffwechselerkrankungen, die durch zu geringe Sekretion endogenen Parathormons entstehen können, gekennzeichnet durch einen Gehalt an 10 bis weniger als 50 µg Gonadoliberin/Einzeldosis oder 1 bis 20 µg eines Gonadoliberinagonisten/Einzeldosis für einen normalgewichtigen Erwachsenen, dadurch gekennzeichnet, daß man Gonadoliberin oder dessen Analogon zusammen mit einem pharmazeutisch unbedenklichen Träger in eine geeignete Zubereitungsform bringt.

7. Verfahren zur Herstellung einer Zubereitung gemäß Anspruch 6 zur Anwendung bei der Behandlung von Hypoparathyreoidismus.

8. Verfahren zur Herstellung einer Zubereitung gemäß Anspruch 6 zur Anwendung bei der Behandlung von Hypocalcämie.

9. Verfahren zur Herstellung einer Zubereitung gemäß Anspruch 6 zur Anwendung bei der Normalisierung des Blutdrucks.

10. Verfahren zur Herstellung einer Zubereitung gemäß Anspruch 6 zur Anwendung bei der Behandlung von Krankheiten, die auf einer zu geringen Glucoseversorgung beruhen.

**Claims** (for the Contracting States : BE, CH, DE, FR, GB, IT, LI, LU, NL, SE)

1. Pharmaceutical formulation for parenteral administration for the treatment of metabolic disorders which may arise due to the secretion of endogenous parathyroid hormone being too low, which contains 0.5 to less than 5 µg of gonadoliberin/single dose, or 0.05 to 0.5 µg of a gonadoliberin agonist/single dose, for an adult of normal weight in a pharmaceutically acceptable vehicle.

2. Formulation as claimed in claim 1 for the treatment of hypoparathyroidism.

3. Formulation as claimed in claim 1 for the treatment of hypocalcemia.

4. Formulation as claimed in claim 1 for the normalization of blood pressure.

5. Formulation as claimed in claim 1 for the treatment of disorders which derive from the glucose supply being too low.

6. Pharmaceutical formulation for administration onto the mucosa for the treatment of metabolic disorders which may arise due to the secretion of endogenous parathyroid hormone being too low, which

contains 10 to less than 50 µg of gonadoliberin/single dose, or 1 to 20 µg of a gonadoliberin agonist/single dose, for an adult of normal weight, in a pharmaceutically acceptable vehicle.

7. Formulation as claimed in claim 6 for the treatment of hypoparathyroidism.

8. Formulation as claimed in claim 6 for the treatment of hypocalcemia.

9. Formulation as claimed in claim 6 for the normalization of blood pressure.

10. Formulation as claimed in claim 6 for the treatment of disorders which derive from the glucose supply being too low.

11. Process for the preparation of a formulation as claimed in one of claims 1 to 10, which comprises converting gonadoliberin or its analog, together with a pharmaceutically acceptable vehicle, into a suitable formulation form.

**Claims** (for the Contracting State AT)

1. Process for the preparation of a pharmaceutical formulation for parenteral use for the treatment of metabolic disorders which may arise due to the secretion of endogenous parathyroid hormone being too low, containing 0.5 to less than 5 µg of gonadoliberin/single dose or 0.05 to 0.5 µg of a gonadoliberin agonist/single dose, for an adult of normal weight, which comprises converting gonadoliberin or its analog, together with a pharmaceutically acceptable vehicle, into a suitable formulation form.

2. Process for the preparation of a formulation as claimed in claim 1 for use for the treatment of hypoparathyroidism.

3. Process for the preparation of a formulation as claimed in claim 1 for use for the treatment of hypocalcemia.

4. Process for the preparation of a formulation as claimed in claim 1 for use for the normalization of blood pressure.

5. Process for the preparation of a formulation as claimed in claim 1 for use for the treatment of disorders which derive from the glucose supply being too low.

6. Process for the preparation of a pharmaceutical formulation for administration onto the mucosa in the treatment of metabolic disorders which may arise due to the secretion of endogenous parathyroid hormone being too low, containing 10 to less than 50 µg of gonadoliberin/single dose, or 1 to 20 µg of a gonadoliberin antagonist/single dose, for an adult of normal weight, which comprises converting gonadoliberin or its analog, together with a pharmaceutically acceptable vehicle, into a suitable formulation form.

7. Process for the preparation of a formulation as claimed in claim 6 for use for the treatment of hypoparathyroidism.

8. Process for the preparation of a formulation as claimed in claim 6 for use for the treatment of hypocalcemia.

9. Process for the preparation of a formulation as claimed in claim 6 for use for the normalization of blood pressure.

10. Process for the preparation of a formulation as claimed in claim 6 for use for the treatment of disorders which derive from the glucose supply being too low.

**Revendications** (pour les Etats contractants : BE, CH, DE, FR, GB, IT, LI, LU, NL, SE)

1. Composition pharmaceutique pour administration parentérale dans le traitement de maladies métaboliques pouvant résulter d'une secrétion trop faible de parathormone endogène, caractérisée par une teneur de 0,5 à moins de 5 µg en gonadolibérine par dose unitaire, ou de 0,05 à 0,5 µg en un agoniste de la gonadolibérine par dose unitaire, pour un sujet adulte de poids normal, dans un véhicule pharmaceutiquement acceptable.

2. Composition selon la revendication 1, pour le traitement de l'hypoparathyroïdie.

3. Composition selon la revendication 1, pour le traitement de l'hypocalcémie.

4. Composition selon la revendication 1, pour le rétablissement d'une pression artérielle normale.

5. Composition selon la revendication 1, pour le traitement de maladies qui sont dues à un trop faible apport de glucose.

6. Composition pharmaceutique pour application sur les muqueuses dans le traitement de maladies métaboliques pouvant résulter d'une secrétion trop faible de parathormone endodogène, caractérisée par une teneur de 10 à moins de 50 µg en gonadolibérine par dose unitaire, ou de 1 à 20 µg en un agoniste de la gonadolibérine par dose unitaire, pour un sujet adulte de poids normal, dans un véhicule pharmaceutiquement acceptable.

7. Composition selon la revendication 6, pour le traitement de l'hypoparathyroïdie.

8. Composition selon la revendication 6, pour le traitement de l'hypocalcémie.

9. Composition selon la revendication 6, pour le rétablissement d'une pression artérielle normale.

10. Composition selon la revendication 6, pour le traitement de maladies qui sont dues à un trop faible apport de glucose.

11. Procédé pour la préparation d'une composition selon l'une des revendications 1 à 10, caractérisé en ce que l'on met sous une forme pharmaceutique appropriée de la gonadolibérine ou un de ses analogues, conjointement avec un véhicule pharmaceutiquement acceptable.

**Revendications** (pour l'Etat contractant AT)

1. Procédé pour la préparation d'une composition pharmaceutique pour administration parentérale dans le traitement de maladies métaboliques pouvant résulter d'une secrétion trop faible de parathormone endogène, ayant une teneur de 0,5 à moins de 5 µg en gonadolibérine par dose unitaire, ou de 0,05 à 0,5 µg en un agoniste de la gonadolibérine par dose unitaire, pour un sujet adulte de poids normal, caractérisé en ce que l'on met sous une forme pharmaceutique appropriée de la gonadolibérine ou un de ses analogues, conjointement avec un véhicule pharmaceutiquement acceptable.
2. Procédé pour la préparation d'une composition selon la revendication 1, à utiliser dans le traitement de l'hypoparathyroïdie.
3. Procédé pour la préparation d'une composition selon la revendication 1, à utiliser dans le traitement de l'hypocalcémie.
4. Procédé pour la préparation d'une composition selon la revendication 1, à utiliser dans le rétablissement d'une pression artérielle normale.
5. Procédé pour la préparation d'une composition selon la revendication 1, à utiliser dans le traitement de maladies qui sont dues à un trop faible apport de glucose.
6. Procédé pour la préparation d'une composition pharmaceutique pour application sur les muqueuses dans le traitement de maladies métaboliques pouvant résulter d'une secrétion trop faible de parathormone endodogène, ayant une teneur de 10 à moins de 50 µg en gonadolibérine par dose unitaire, ou de 1 à 20 µg en un agoniste de la gonadolibérine par dose unitaire, pour un sujet adulte de poids normal, caractérisé en ce que l'on met sous une forme pharmaceutique appropriée de la gonadolibérine ou un de ses analogues, conjointement avec un véhicule pharmaceutiquement acceptable.
7. Procédé pour la préparation d'une composition selon la revendication 6, à utiliser dans le traitement de l'hypoparathyroïdie.
8. Procédé pour la préparation d'une composition selon la revendication 6, à utiliser dans le traitement de l'hypocalcémie.
9. Procédé pour la préparation d'une composition selon la revendication 6, à utiliser dans le rétablissement d'une pression artérielle normale.
10. Procédé pour la préparation d'une composition selon la revendication 6, à utiliser dans le traitement de maladies qui sont dues à un trop faible apport de glucose.

FIG. 1

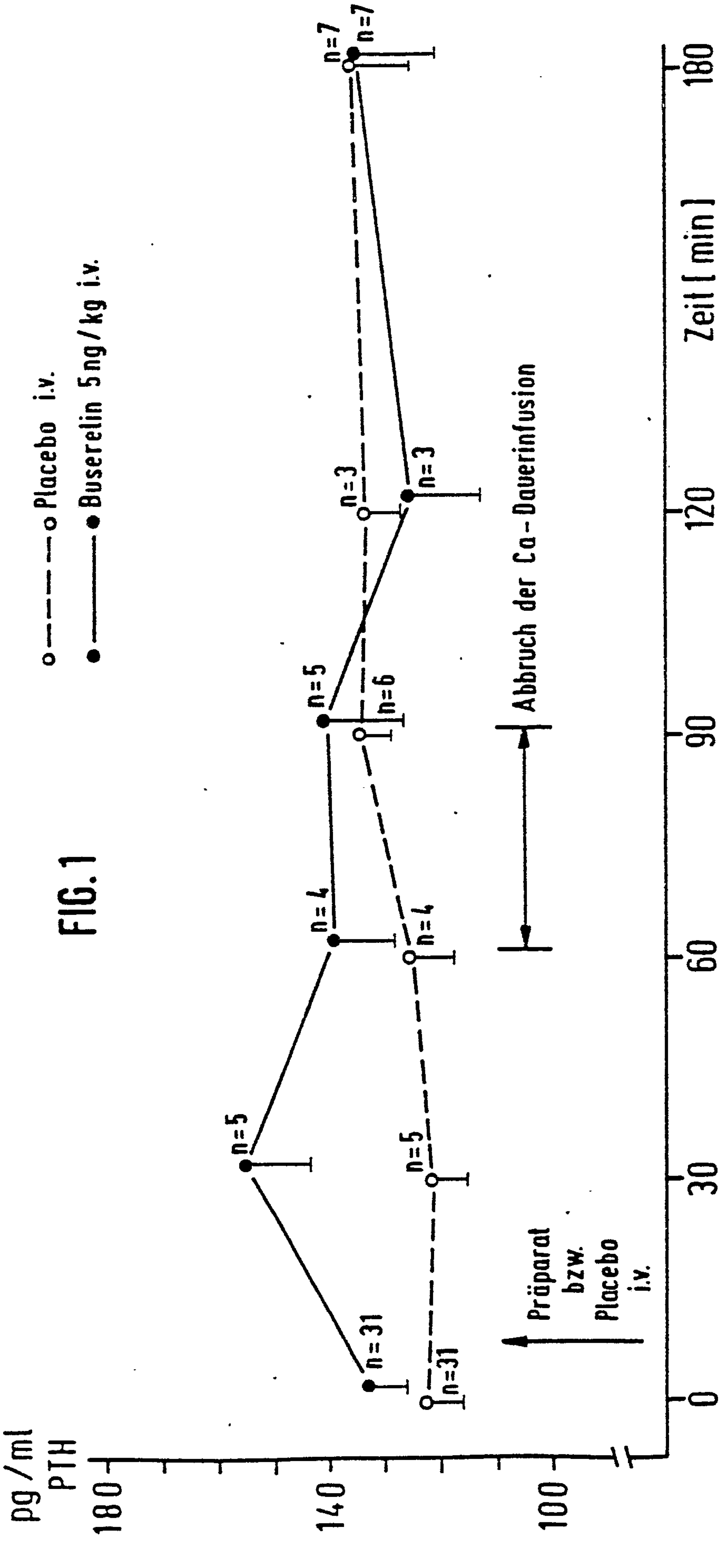

pg/ml PTH
180
140
100
0
30
60
90
120
180
Zeit [min]
Placebo i.v.
Buserelin 5 ng/kg i.v.
Abbruch der Ca-Dauerinfusion
Präparat bzw. Placebo i.v.
n=31
n=31
n=5
n=5
n=4
n=4
n=5
n=6
n=3
n=3
n=7
n=7

FIG. 2

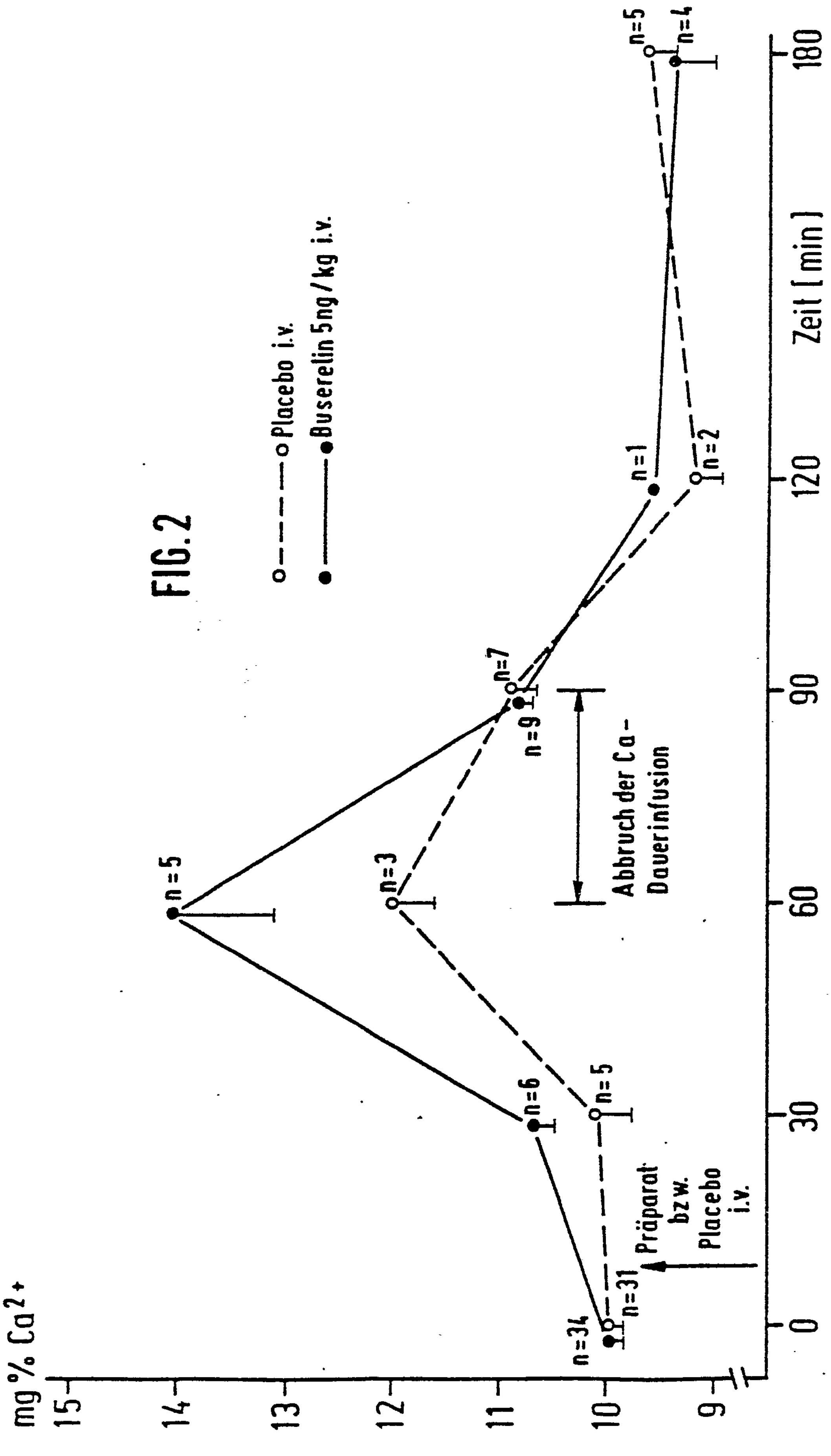
Placebo i.v.
Buserelin 5ng/kg i.v.
mg % Ca2+
15
14
13
12
11
10
9
0
30
60
90
120
180
Zeit [min]
Präparat bzw. Placebo i.v.
Abbruch der Ca-Dauerinfusion
n=34
n=31
n=6
n=5
n=5
n=3
n=9
n=7
n=1
n=2
n=5
n=4

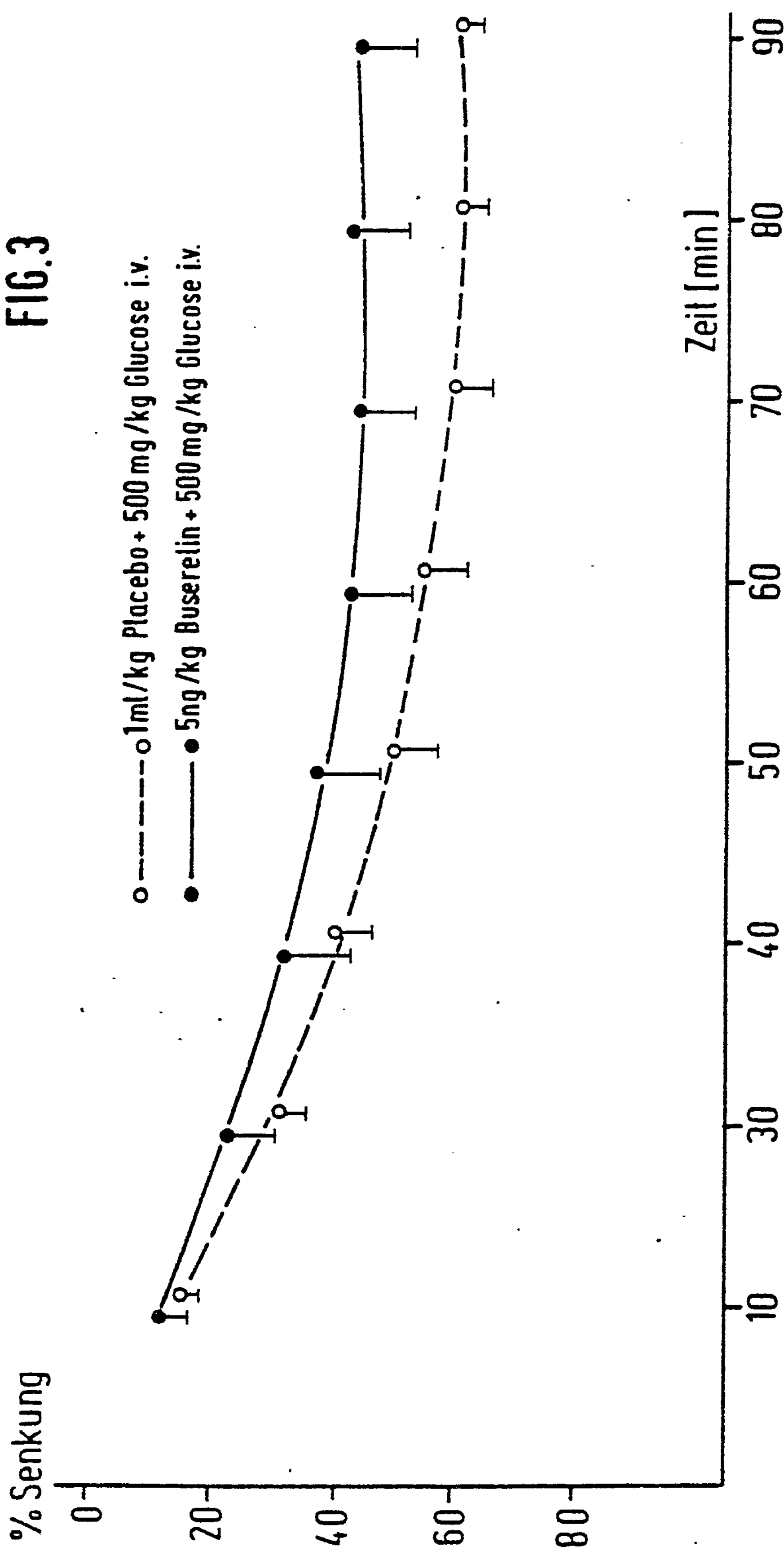
o----o 1ml/kg Placebo + 500 mg/kg Glucose i.v.
●——● 5ng/kg Buserelin + 500 mg/kg Glucose i.v.
% Senkung
0
20
40
60
80
10
30
40
50
60
70
80
90
Zeit [min]

FIG. 3